# EUROPEAN PATENT APPLICATION

(11) **EP 2 554 182 A1**
(43) Date of publication of application: **06.02.2013**
(21) Application number: 11382269.6
(22) Date of filing: 03.08.2011
(51) Int. Cl.: A61K 38/17, A61P 21/06, C12N 15/864

(54) **Mammal sestrins in the treatment of muscle wasting**

(71) Applicant: Universitat Pompeu-Fabra, 08002 Barcelona (ES); Institució Catalana de Recerca i Estudis Avançats (ICREA), 08010 Barcelona (ES)
(72) Inventor: Munoz Canoves, Purificación, 08003 BARCELONA (ES); Raya Chamorro, Marina, 08003 BARCELONA (ES); Perdiguero Santamaria, Eusebio, 08003 BARCELONA (ES); Luis Serrano, Antonio, 08003 BARCELONA (ES); Sousa Corado Victor, Pedro Miguel, 08003 BARCELONA (ES)
(74) Representative: ZBM Patents

(57) **Abstract**

The invention discloses a mammal sestrin product applicable in muscle atrophy, as well as any recombinant construction (vectors) comprising this mammal sestrin. A non-human animal model of muscle cell hypertrophy is also disclosed. In particular, the invention proposes tools for the treatment of atrophies of any aetiology, such as muscular dystrophies, cachexia, and atrophy related to malnutrition or to immobilization.

SEQUENCE LISTING

<110> UNIVERSITAT POMPEU FABRA
      INSTITUCIÓ CATALANA DE RECERCA I ESTUDIS AVANÇATS

<120> Mammal sestrins in the treatment of muscle wasting

<130> P2016EP00

<160> 7

<170> PatentIn version 3.5

<210> 1
<211> 492
<212> PRT
<213> Homo sapiens

<400> 1

Met Arg Leu Ala Ala Ala Ala Asn Glu Ala Tyr Thr Ala Pro Leu Ala
1               5                   10                  15

Val Ser Gly Leu Leu Gly Cys Lys Gln Cys Gly Gly Gly Arg Asp Gln
            20                  25                  30

Asp Glu Glu Leu Gly Ile Arg Ile Pro Arg Pro Leu Gly Gln Gly Pro
        35                  40                  45

Ser Arg Phe Ile Pro Glu Lys Glu Ile Leu Gln Val Gly Ser Glu Asp
    50                  55                  60

Ala Gln Met His Ala Leu Phe Ala Asp Ser Phe Ala Ala Leu Gly Arg
65                  70                  75                  80

Leu Asp Asn Ile Thr Leu Val Met Val Phe His Pro Gln Tyr Leu Glu
                85                  90                  95

Ser Phe Leu Lys Thr Gln His Tyr Leu Leu Gln Met Asp Gly Pro Leu
            100                 105                 110

Pro Leu His Tyr Arg His Tyr Ile Gly Ile Met Ala Ala Ala Arg His
        115                 120                 125

Gln Cys Ser Tyr Leu Val Asn Leu His Val Asn Asp Phe Leu His Val
    130                 135                 140

Gly Gly Asp Pro Lys Trp Leu Asn Gly Leu Glu Asn Ala Pro Gln Lys
145                 150                 155                 160

Leu Gln Asn Leu Gly Glu Leu Asn Lys Val Leu Ala His Arg Pro Trp
                165                 170                 175

Leu Ile Thr Lys Glu His Ile Glu Gly Leu Leu Lys Ala Glu Glu His
            180                 185                 190

Ser Trp Ser Leu Ala Glu Leu Val His Ala Val Val Leu Leu Thr His
        195                 200                 205

Tyr His Ser Leu Ala Ser Phe Thr Phe Gly Cys Gly Ile Ser Pro Glu
    210                 215                 220

Ile His Cys Asp Gly Gly His Thr Phe Arg Pro Pro Ser Val Ser Asn
225                 230                 235                 240

Tyr Cys Ile Cys Asp Ile Thr Asn Gly Asn His Ser Val Asp Glu Met
                245                 250                 255

Pro Val Asn Ser Ala Glu Asn Val Ser Val Ser Asp Ser Phe Phe Glu
            260                 265                 270

Val Glu Ala Leu Met Glu Lys Met Arg Gln Leu Gln Glu Cys Arg Asp
        275                 280                 285

Glu Glu Glu Ala Ser Gln Glu Glu Met Ala Ser Arg Phe Glu Ile Glu
    290                 295                 300

Lys Arg Glu Ser Met Phe Val Phe Ser Ser Asp Asp Glu Glu Val Thr
305                 310                 315                 320

Pro Ala Arg Ala Val Ser Arg His Phe Glu Asp Thr Ser Tyr Gly Tyr
                325                 330                 335

Lys Asp Phe Ser Arg His Gly Met His Val Pro Thr Phe Arg Val Gln
            340                 345                 350

Asp Tyr Cys Trp Glu Asp His Gly Tyr Ser Leu Val Asn Arg Leu Tyr
        355                 360                 365

Pro Asp Val Gly Gln Leu Ile Asp Glu Lys Phe His Ile Ala Tyr Asn
    370                 375                 380

Leu Thr Tyr Asn Thr Met Ala Met His Lys Asp Val Asp Thr Ser Met
385                 390                 395                 400

Leu Arg Arg Ala Ile Trp Asn Tyr Ile His Cys Met Phe Gly Ile Arg
                405                 410                 415

Tyr Asp Asp Tyr Asp Tyr Gly Glu Ile Asn Gln Leu Leu Asp Arg Ser
            420                 425                 430

Phe Lys Val Tyr Ile Lys Thr Val Val Cys Thr Pro Glu Lys Val Thr
        435                 440                 445

Lys Arg Met Tyr Asp Ser Phe Trp Arg Gln Phe Lys His Ser Glu Lys
    450                 455                 460

Val His Val Asn Leu Leu Leu Ile Glu Ala Arg Met Gln Ala Glu Leu
465                 470                 475                 480

Leu Tyr Ala Leu Arg Ala Ile Thr Arg Tyr Met Thr
                485                 490


<210>  2
<211>  480
<212>  PRT
<213>  Homo sapiens

<400>  2

Met Ile Val Ala Asp Ser Glu Cys Arg Ala Glu Leu Lys Asp Tyr Leu
1               5                   10                  15

Arg Phe Ala Pro Gly Gly Val Gly Asp Ser Gly Pro Gly Glu Glu Gln
            20                  25                  30

Arg Glu Ser Arg Ala Arg Arg Gly Pro Arg Gly Pro Ser Ala Phe Ile
        35                  40                  45

Pro Val Glu Glu Val Leu Arg Glu Gly Ala Glu Ser Leu Glu Gln His
    50                  55                  60

Leu Gly Leu Glu Ala Leu Met Ser Ser Gly Arg Val Asp Asn Leu Ala
65                  70                  75                  80

Val Val Met Gly Leu His Pro Asp Tyr Phe Thr Ser Phe Trp Arg Leu
                85                  90                  95

His Tyr Leu Leu Leu His Thr Asp Gly Pro Leu Ala Ser Ser Trp Arg
            100                 105                 110

His Tyr Ile Ala Ile Met Ala Ala Ala Arg His Gln Cys Ser Tyr Leu

115                 120                 125

Val Gly Ser His Met Ala Glu Phe Leu Gln Thr Gly Gly Asp Pro Glu
    130                 135                 140

Trp Leu Leu Gly Leu His Arg Ala Pro Glu Lys Leu Arg Lys Leu Ser
145                 150                 155                 160

Glu Ile Asn Lys Leu Leu Ala His Arg Pro Trp Leu Ile Thr Lys Glu
                165                 170                 175

His Ile Gln Ala Leu Leu Lys Thr Gly Glu His Thr Trp Ser Leu Ala
            180                 185                 190

Glu Leu Ile Gln Ala Leu Val Leu Leu Thr His Cys His Ser Leu Ser
        195                 200                 205

Ser Phe Val Phe Gly Cys Gly Ile Leu Pro Glu Gly Asp Ala Asp Gly
    210                 215                 220

Ser Pro Ala Pro Gln Ala Pro Thr Pro Pro Ser Glu Gln Ser Ser Pro
225                 230                 235                 240

Pro Ser Arg Asp Pro Leu Asn Asn Ser Gly Gly Phe Glu Ser Ala Arg
                245                 250                 255

Asp Val Glu Ala Leu Met Glu Arg Met Gln Gln Leu Gln Glu Ser Leu
            260                 265                 270

Leu Arg Asp Glu Gly Thr Ser Gln Glu Glu Met Glu Ser Arg Phe Glu
        275                 280                 285

Leu Glu Lys Ser Glu Ser Leu Leu Val Thr Pro Ser Ala Asp Ile Leu
    290                 295                 300

Glu Pro Ser Pro His Pro Asp Met Leu Cys Phe Val Glu Asp Pro Thr
305                 310                 315                 320

Phe Gly Tyr Glu Asp Phe Thr Arg Arg Gly Ala Gln Ala Pro Pro Thr
                325                 330                 335

Phe Arg Ala Gln Asp Tyr Thr Trp Glu Asp His Gly Tyr Ser Leu Ile
            340                 345                 350

Gln Arg Leu Tyr Pro Glu Gly Gly Gln Leu Leu Asp Glu Lys Phe Gln

355                 360                 365

Ala Ala Tyr Ser Leu Thr Tyr Asn Thr Ile Ala Met His Ser Gly Val
    370                 375                 380

Asp Thr Ser Val Leu Arg Arg Ala Ile Trp Asn Tyr Ile His Cys Val
385                 390                 395                 400

Phe Gly Ile Arg Tyr Asp Asp Tyr Asp Tyr Gly Glu Val Asn Gln Leu
                405                 410                 415

Leu Glu Arg Asn Leu Lys Val Tyr Ile Lys Thr Val Ala Cys Tyr Pro
            420                 425                 430

Glu Lys Thr Thr Arg Arg Met Tyr Asn Leu Phe Trp Arg His Phe Arg
        435                 440                 445

His Ser Glu Lys Val His Val Asn Leu Leu Leu Leu Glu Ala Arg Met
    450                 455                 460

Gln Ala Ala Leu Leu Tyr Ala Leu Arg Ala Ile Thr Arg Tyr Met Thr
465                 470                 475                 480

<210>  3
<211>  492
<212>  PRT
<213>  Homo sapiens

<400>  3

Met Asn Arg Gly Gly Gly Ser Pro Ser Ala Ala Ala Asn Tyr Leu Leu
1               5                   10                  15

Cys Thr Asn Cys Arg Lys Val Leu Arg Lys Asp Lys Arg Ile Arg Val
            20                  25                  30

Ser Gln Pro Leu Thr Arg Gly Pro Ser Ala Phe Ile Pro Glu Lys Glu
        35                  40                  45

Val Val Gln Ala Asn Thr Val Asp Glu Arg Thr Asn Phe Leu Val Glu
    50                  55                  60

Glu Tyr Ser Thr Ser Gly Arg Leu Asp Asn Ile Thr Gln Val Met Ser
65                  70                  75                  80

Leu His Thr Gln Tyr Leu Glu Ser Phe Leu Arg Ser Gln Phe Tyr Met
                85                  90                  95

Leu Arg Met Asp Gly Pro Leu Pro Leu Pro Tyr Arg His Tyr Ile Ala
            100                 105                 110

Ile Met Ala Ala Ala Arg His Gln Cys Ser Tyr Leu Ile Asn Met His
        115                 120                 125

Val Asp Glu Phe Leu Lys Thr Gly Gly Ile Ala Glu Trp Leu Asn Gly
    130                 135                 140

Leu Glu Tyr Val Pro Gln Arg Leu Lys Asn Leu Asn Glu Ile Asn Lys
145                 150                 155                 160

Leu Leu Ala His Arg Pro Trp Leu Ile Thr Lys Glu His Ile Gln Lys
                165                 170                 175

Leu Val Lys Thr Gly Glu Asn Asn Trp Ser Leu Pro Glu Leu Val His
            180                 185                 190

Ala Val Val Leu Leu Ala His Tyr His Ala Leu Ala Ser Phe Val Phe
        195                 200                 205

Gly Ser Gly Ile Asn Pro Glu Arg Asp Pro Glu Ile Ser Asn Gly Phe
    210                 215                 220

Arg Leu Ile Ser Val Asn Asn Phe Cys Val Cys Asp Leu Ala Asn Asp
225                 230                 235                 240

Asn Asn Ile Glu Asn Ala Ser Leu Ser Gly Ser Asn Phe Gly Ile Val
                245                 250                 255

Asp Ser Leu Ser Glu Leu Glu Ala Leu Met Glu Arg Met Lys Arg Leu
            260                 265                 270

Gln Glu Glu Arg Glu Asp Glu Glu Ala Ser Gln Glu Glu Met Ser Thr
        275                 280                 285

Arg Phe Glu Lys Glu Lys Lys Glu Ser Leu Phe Val Val Ser Gly Asp
    290                 295                 300

Thr Phe His Ser Phe Pro His Ser Asp Phe Glu Asp Asp Met Ile Ile
305                 310                 315                 320

Thr Ser Asp Val Ser Arg Tyr Ile Glu Asp Pro Gly Phe Gly Tyr Glu
                325                 330                 335

Asp Phe Ala Arg Arg Gly Glu Glu His Leu Pro Thr Phe Arg Ala Gln
            340                 345                 350

Asp Tyr Thr Trp Glu Asn His Gly Phe Ser Leu Val Asn Arg Leu Tyr
        355                 360                 365

Ser Asp Ile Gly His Leu Leu Asp Glu Lys Phe Arg Met Val Tyr Asn
    370                 375                 380

Leu Thr Tyr Asn Thr Met Ala Thr His Glu Asp Val Asp Thr Thr Met
385                 390                 395                 400

Leu Arg Arg Ala Leu Phe Asn Tyr Val His Cys Met Phe Gly Ile Arg
                405                 410                 415

Tyr Asp Asp Tyr Asp Tyr Gly Glu Val Asn Gln Leu Leu Glu Arg Ser
            420                 425                 430

Leu Lys Val Tyr Ile Lys Thr Val Thr Cys Tyr Pro Glu Arg Thr Thr
        435                 440                 445

Lys Arg Met Tyr Asp Ser Tyr Trp Arg Gln Phe Lys His Ser Glu Lys
    450                 455                 460

Val His Val Asn Leu Leu Leu Met Glu Ala Arg Met Gln Ala Glu Leu
465                 470                 475                 480

Leu Tyr Ala Leu Arg Ala Ile Thr Arg His Leu Thr
                485                 490

<210>  4
<211>  492
<212>  PRT
<213>  Mus musculus

<400>  4

Met Arg Leu Ala Ala Ala Ser Asn Glu Ala Tyr Ala Ala Ser Leu Ala
1               5                   10                  15

Val Ser Glu Leu Leu Ser Cys His Gln Cys Gly Gly Asp Arg Gly Gln
            20                  25                  30

Asp Glu Glu Leu Gly Ile Arg Ile Pro Arg Pro Leu Gly His Gly Pro
        35                  40                  45

Ser Arg Phe Ile Pro Glu Lys Glu Met Leu Gln Val Gly Ser Glu Asp
    50                  55                  60

Ala Gln Met His Ala Leu Phe Ala Asp Ser Phe Ala Ala Leu Gly Arg
65                  70                  75                  80

Leu Asp Asn Ile Thr Leu Val Met Val Phe His Pro Gln Tyr Leu Glu
                85                  90                  95

Ser Phe Leu Lys Thr Gln His Tyr Leu Leu Gln Met Asp Gly Pro Leu
            100                 105                 110

Pro Leu His Tyr Arg His Tyr Ile Gly Ile Met Ala Ala Ala Arg His
        115                 120                 125

Gln Cys Ser Tyr Leu Val Asn Leu His Val Ser Asp Phe Leu His Val
    130                 135                 140

Gly Gly Asp Pro Lys Trp Leu Asn Gly Leu Glu Asn Ala Pro Gln Lys
145                 150                 155                 160

Leu Gln Asn Leu Gly Glu Leu Asn Lys Val Leu Ala His Arg Pro Trp
                165                 170                 175

Leu Ile Thr Lys Glu His Ile Glu Gly Leu Leu Lys Ala Glu Glu His
            180                 185                 190

Ser Trp Ser Leu Ala Glu Leu Val His Ala Val Val Leu Leu Thr His
        195                 200                 205

Tyr His Ser Leu Ala Ser Phe Thr Phe Gly Cys Gly Ile Ser Pro Glu
    210                 215                 220

Ile His Cys Asp Gly Gly His Thr Phe Arg Pro Pro Ser Val Ser Asn
225                 230                 235                 240

Tyr Cys Ile Cys Asp Ile Thr Asn Gly Asn His Ser Val Asp Glu Met
                245                 250                 255

Gln Val Asn Ser Ala Gly Asn Ala Ser Val Ser Asp Ser Phe Phe Glu
            260                 265                 270

Val Glu Ala Leu Met Glu Lys Met Arg Gln Leu Gln Glu Cys Arg Glu
        275                 280                 285

Glu Glu Glu Ala Ser Gln Glu Glu Met Ala Ser Arg Phe Glu Met Glu
    290                 295                 300

Lys Arg Glu Ser Met Phe Val Phe Ser Ser Asp Asp Asp Glu Val Thr
305                 310                 315                 320

Pro Ala Arg Asp Val Ser Arg His Phe Glu Asp Thr Ser Tyr Gly Tyr
                325                 330                 335

Lys Asp Phe Ser Arg His Gly Met His Val Pro Thr Phe Arg Val Gln
            340                 345                 350

Asp Tyr Cys Trp Glu Asp His Gly Tyr Ser Leu Val Asn Arg Leu Tyr
        355                 360                 365

Pro Asp Val Gly Gln Leu Ile Asp Glu Lys Phe His Ile Pro Tyr Asn
    370                 375                 380

Leu Thr Tyr Asn Thr Met Ala Met His Lys Asp Val Asp Thr Ser Met
385                 390                 395                 400

Leu Arg Arg Ala Ile Trp Asn Tyr Ile His Cys Met Phe Gly Ile Arg
                405                 410                 415

Tyr Asp Asp Tyr Asp Tyr Gly Glu Ile Asn Gln Leu Leu Asp Arg Ser
            420                 425                 430

Phe Lys Val Tyr Ile Lys Thr Val Val Cys Thr Pro Glu Lys Val Thr
        435                 440                 445

Lys Arg Met Tyr Asp Ser Phe Trp Arg Gln Phe Lys His Ser Glu Lys
    450                 455                 460

Val His Val Asn Leu Leu Leu Ile Glu Ala Arg Met Gln Ala Glu Leu
465                 470                 475                 480

Leu Tyr Ala Leu Arg Ala Ile Thr Arg Tyr Met Thr
                485                 490

<210>  5
<211>  480
<212>  PRT
<213>  Mus musculus

<400>  5

Met Ile Val Ala Asp Ser Glu Cys His Ser Glu Ile Lys Gly Tyr Leu
1               5                   10                  15

Pro Phe Thr Arg Gly Gly Val Ala Gly Pro Glu Thr Arg Glu Glu His
            20                  25                  30

Arg Glu Gly Gln Ala Arg Arg Gly Ser Arg Gly Pro Ser Ala Phe Ile
        35                  40                  45

Pro Val Glu Glu Ile Leu Arg Glu Gly Ala Glu Ser Leu Glu Gln His
    50                  55                  60

Leu Gly Leu Glu Ala Leu Met Ser Ser Gly Arg Val Asp Asn Leu Ala
65                  70                  75                  80

Val Val Met Gly Leu His Pro Asp Tyr Leu Ser Ser Phe Trp Arg Leu
                85                  90                  95

His Tyr Leu Leu Leu His Thr Asp Gly Pro Leu Ala Ser Ser Trp Arg
            100                 105                 110

His Tyr Ile Ala Ile Met Ala Ala Ala Arg His Gln Cys Ser Tyr Leu
        115                 120                 125

Val Gly Ser His Met Thr Glu Phe Leu Gln Thr Gly Gly Asp Pro Glu
    130                 135                 140

Trp Leu Leu Gly Leu His Arg Ala Pro Glu Lys Leu Arg Lys Leu Ser
145                 150                 155                 160

Glu Val Asn Lys Leu Leu Ala His Arg Pro Trp Leu Ile Thr Lys Glu
                165                 170                 175

His Ile Gln Ala Leu Leu Lys Thr Gly Glu His Ser Trp Ser Leu Ala
            180                 185                 190

Glu Leu Ile Gln Ala Leu Val Leu Leu Thr His Cys His Ser Leu Ala
        195                 200                 205

Ser Phe Val Phe Gly Cys Gly Ile Leu Pro Glu Gly Asp Ala Glu Gly
    210                 215                 220

Ser Pro Ala Ser Gln Ala Pro Ser Pro Pro Ser Glu Gln Gly Thr Pro
225                 230                 235                 240

Pro Ser Gly Asp Pro Leu Asn Asn Ser Gly Gly Phe Glu Ala Ala Arg
                245                 250                 255

Asp Val Glu Ala Leu Met Glu Arg Met Arg Gln Leu Gln Glu Ser Leu
            260                 265                 270

Leu Arg Asp Glu Gly Ala Ser Gln Glu Glu Met Glu Asn Arg Phe Glu
        275                 280                 285

Leu Glu Lys Ser Glu Ser Leu Leu Val Thr Pro Ser Ala Asp Ile Leu
    290                 295                 300

Glu Pro Ser Pro His Pro Asp Ile Leu Cys Phe Val Glu Asp Pro Ala
305                 310                 315                 320

Phe Gly Tyr Glu Asp Phe Thr Arg Arg Gly Thr Gln Ala Pro Pro Thr
                325                 330                 335

Phe Arg Ala Gln Asp Tyr Thr Trp Glu Asp His Gly Tyr Ser Leu Ile
            340                 345                 350

Gln Arg Leu Tyr Pro Glu Gly Gly Gln Leu Leu Asp Glu Lys Phe Gln
        355                 360                 365

Val Ala Cys Ser Leu Thr Tyr Asn Thr Ile Ala Met His Ser Gly Val
    370                 375                 380

Asp Thr Ser Met Leu Arg Arg Ala Ile Trp Asn Tyr Ile His Cys Val
385                 390                 395                 400

Phe Gly Ile Arg Tyr Asp Asp Tyr Asp Tyr Gly Glu Val Asn Gln Leu
                405                 410                 415

Leu Glu Arg Asn Leu Lys Ile Tyr Ile Lys Thr Val Ala Cys Tyr Pro
            420                 425                 430

Glu Lys Thr Thr Arg Arg Met Tyr Asn Leu Phe Trp Arg His Phe Arg
        435                 440                 445

His Ser Glu Lys Val His Val Asn Leu Leu Leu Leu Glu Ala Arg Met
    450                 455                 460

Gln Ala Ala Leu Leu Tyr Ala Leu Arg Ala Ile Thr Arg Tyr Met Thr
465                 470                 475                 480

<210>  6
<211>  492
<212>  PRT
<213>  Mus musculus

<400>  6

Met Asn Arg Gly Gly Ser Ser Ala Ser Ala Ser Ala Asn Tyr Leu Leu
1               5                   10                  15

Cys Thr Asn Cys Arg Lys Val Leu Arg Lys Asp Lys Arg Ile Arg Val
            20                  25                  30

Ser Gln Pro Leu Thr Arg Gly Pro Ser Ala Phe Ile Pro Glu Lys Glu
        35                  40                  45

Val Val Gln Ala Asn Thr Ala Asp Glu Arg Thr Asn Phe Leu Val Glu
    50                  55                  60

Glu Tyr Ser Thr Ser Gly Arg Leu Asp Asn Ile Thr Gln Val Met Ser
65                  70                  75                  80

Leu His Thr Gln Tyr Leu Glu Ser Phe Leu Arg Ser Gln Phe Tyr Met
                85                  90                  95

Leu Arg Met Asp Gly Pro Leu Pro Leu Pro Asp Arg His Tyr Ile Ala
            100                 105                 110

Ile Met Ala Ala Ala Arg His Gln Cys Ser Tyr Leu Ile Asn Met His
        115                 120                 125

Val Asp Glu Phe Leu Lys Thr Gly Gly Ile Ala Glu Trp Leu Asn Gly
    130                 135                 140

Leu Glu Tyr Val Pro Gln Arg Leu Arg Asn Leu Asn Glu Ile Asn Lys
145                 150                 155                 160

Leu Leu Ala His Arg Pro Trp Leu Ile Thr Lys Glu His Ile Gln Lys
                165                 170                 175

Leu Val Lys Thr Gly Glu Asn Asn Trp Ser Leu Pro Glu Leu Val His
            180                 185                 190

Ala Val Val Leu Leu Ala His Tyr His Ala Leu Ala Ser Phe Val Phe
        195                 200                 205

Gly Ser Gly Ile Asn Pro Glu Arg Asp Pro Gly Ile Ala Asn Gly Phe

210                 215                 220

Arg Leu Ile Ser Val Ser Ser Phe Cys Val Cys Asp Leu Ala Asn Asp
225                 230                 235                 240

Asn Ser Ile Glu Asn Thr Ser Leu Ala Gly Ser Asn Phe Gly Ile Val
                245                 250                 255

Asp Ser Leu Gly Glu Leu Glu Ala Leu Met Glu Arg Met Lys Arg Leu
            260                 265                 270

Gln Glu Asp Arg Glu Asp Asp Glu Thr Thr Arg Glu Glu Met Thr Thr
        275                 280                 285

Arg Phe Glu Lys Glu Lys Lys Glu Ser Leu Phe Val Val Pro Gly Glu
    290                 295                 300

Thr Leu His Ala Phe Pro His Ser Asp Phe Glu Asp Asp Val Ile Val
305                 310                 315                 320

Thr Ala Asp Val Ser Arg Tyr Ile Glu Asp Pro Ser Phe Gly Tyr Glu
                325                 330                 335

Asp Phe Ala Arg Arg Gly Glu Glu His Leu Pro Thr Phe Arg Ala Gln
            340                 345                 350

Asp Tyr Thr Trp Glu Asn His Gly Phe Ser Leu Val Asn Arg Leu Tyr
        355                 360                 365

Ser Asp Ile Gly His Leu Leu Asp Glu Lys Phe Arg Met Val Tyr Asn
    370                 375                 380

Leu Thr Tyr Asn Thr Met Ala Thr His Glu Asp Val Asp Thr Thr Thr
385                 390                 395                 400

Leu Arg Arg Ala Leu Phe Asn Tyr Val His Cys Met Phe Gly Ile Arg
                405                 410                 415

Tyr Asp Asp Tyr Asp Tyr Gly Glu Val Asn Gln Leu Leu Glu Arg Ser
            420                 425                 430

Leu Lys Val Tyr Ile Lys Thr Val Thr Cys Tyr Pro Glu Arg Thr Thr
        435                 440                 445

Lys Arg Met Tyr Asp Ser Tyr Trp Arg Gln Phe Thr His Ser Glu Lys

450                 455                 460

Val His Val Asn Leu Leu Leu Met Glu Ala Arg Met Gln Ala Glu Leu
465                 470                 475                 480

Leu Tyr Ala Leu Arg Ala Ile Thr Arg His Leu Thr
                485                 490

<210>  7
<211>  21
<212>  DNA
<213>  Artificial

<220>
<223>   Small-hairpin RNA for depleting expression Mus musculus
       sestrin-1

<400>  7
gttcaagcac tctgagaagg t                                                21

## Description

The present invention is related to the field of medicine and, especially, the field of muscle disorders or myopathies. More particularly, the invention is related to new agents for the treatment of muscular atrophy or wasting.

### BACKGROUND ART

Muscle atrophy, also called muscle wasting, is defined as a decrease in the mass of the muscle; it can be a partial or complete wasting away of muscle. Atrophic muscle is a weak muscle, due to the fact that the ability to exert force is related to mass. Muscle wasting or atrophy refers then to the progressive loss of muscle mass and/or to the progressive weakening and degeneration of the skeletal or voluntary muscles, which control movement, or of the cardiac muscles, which control the heart (cardiomyopathies), and smooth muscles.

Physiologically, muscle atrophy occurs by an imbalance between protein synthesis and protein degradation. During atrophy, there is a down-regulation of protein synthesis pathways, and an activation of protein breakdown pathways. Muscle wasting is associated to a catabolic state. The term "catabolic state" means a pathological situation in which the catabolic pathways are activated, such as hepatic glycogenolysis, lipolysis, proteolysis and thermogenesis.

The particular protein degradation pathway which seems to be responsible for much of the muscle loss seen in a muscle undergoing atrophy is the ATP-dependent ubiquitin/proteasome pathway. In this system, specific proteins are targeted for destruction by the ligation of at least four copies of a small peptide called ubiquitin onto a substrate protein. When a substrate is thus "poly-ubiquitinated", it is targeted for destruction by the proteasome. Particular enzymes in the ubiquitin/proteasome pathway allow ubiquitination to be directed to some proteins but not others. A search for transcriptional markers of muscle atrophy led to the discovery of the E3 ubiquitin ligases MuRF1 and MAFbx (also named Atrogin-1). Each E3 ubiquitin ligase binds to a particular set of substrates, causing their ubiquitination.

Muscle atrophy is a highly debilitating response associated to a wide range of diseases or physical conditions or states, including among others muscular dystrophies, such as Duchene's muscular dystrophy, Becker's muscular dystrophy, limb girdle muscular dystrophy, etc., post-polio muscle atrophy, cachexia, such as cardiac disease, AIDS and cancer associated cachexia, leprosy, diabetes, cancer, renal disease, obstructive pulmonary disease, emphysema, paraplegia, hemiplegia, and sarcopenia. Among those physical states or conditions leading also to muscle atrophy or wasting, malnutrition, immobilized or casted limb, long term hospitalization and non or reduced gravity situations as space trips are included.

Especially of interest is the cachexia, which is defined as wasting of lean body mass that cannot be nutritionally reversed, thus indicating a deregulation in the pathways maintaining body composition. Cachexia is observed as a co-lateral symptom in cancers, HIV-virus infections and malnutrition states. It is a positive risk factor for death, meaning that if the patient has cachexia, the chance of death from the underlying condition is dramatically increased.

Current palliative treatments of muscle atrophy associated disorders or physical conditions include the use of glucocorticoids, which have demonstrated a slow muscle strength declination in cases of Duchene's Muscular Distrophy (DMD). In general, muscular dystrophy refers to a group of hereditary muscle diseases that weakens the muscles that move the human body. Muscular dystrophies are characterized by progressive skeletal muscle weakness, defects in muscle proteins, and the final death of muscle cells and tissue.

Focusing on the mechanism involved in muscle dystrophies, there have been proposed some putative therapeutically approaches. Rüegg et al., "Molecular Mechanisms and Treatment Options for Muscle Wasting Diseases", Annu. Rev. Pharmacol. Toxicol - 2011, Vol. N° 51, pp.: 373-395, propose the use of Insulin-Like Growth Factor 1 (IGF-1) in muscle dystrophies. The proposal is based on the rationale that activation of the IGF-1 pathway not only leads to a compensatory hypertrophy, but also affects pathways whose function is lost in muscle dystrophies. One of these pathways is the one in which E3 ubiquitin ligases MuRF1 and MAFbx are involved.

The IGF-1 can block the transcriptional upregulation of MuRF1 and MAFbx via the phosphatidylinositol-3 kinase (Pl3k)/Akt/Foxo pathway. MuRF1's substrates include several components of the sarcomeric thick filament, including myosin heavy chain. Thus, by blocking MuRF1, IGF-1 prevents the breakdown of the thick filament, particularly myosin heavy chain, which is asymmetrically lost in settings of cortisol-linked skeletal muscle atrophy. IGF-1/Pl3K/Akt signalling also dominantly inhibits the effects of myostatin, which is a member of the transforming growth factor-b family of proteins.

Nonetheless, Rüegg et al. *(supra)* also indicate that still many challenges have to be faced before using IGF-1 in muscular dystrophies. First of all, IGF-1 is expressed in several isoforms, and it is not clear whether all are equally potent in skeletal muscle. Further, IGF-1 released into the blood is bound to many proteins. IGF-1 is also involved in the control of cell and organ size systemically, making its use problematic for the patients who already have normal levels of IGF-1. Finally, high levels of IGF-1 can cause hypoglycaemia by stimulating the insulin receptor.

In US 20060069049 it is indicated the use of agents capable of regulating the phosphorylation and activity of the transcription factor Foxo, which is a protein that upregulates MuRF1 and MAFbx.

On the other hand, those individuals suffering from cachexia (of any of the possible aetiologies) are currently treated with corticosteroids or drugs that mimic progesterone in order to increase the appetite and reverse weight loss. Nonetheless, these compounds are not able to reverse muscle loss.

It is then clear from the above-mentioned therapeutically strategies to face atrophic or wasted muscles, that there is still a need for alternative therapies or more effective approaches to fight all those diseases and physical conditions involving muscular atrophy.

### SUMMARY OF THE INVENTION

It has been surprisingly found that administering to mice an adeno-associated vector comprising mammal sestrins, which sestrins are then over-expressed into the mice, promote muscle cell hypertrophy and allows rescuing atrophic cells.

Thus, in a first aspect the invention provides a mammal sestrin product for use in the treatment of muscle atrophy. This aspect can be formulated as the use of a mammal sestrin product for the manufacture of a medicament for the treatment of muscular atrophy. The invention also provides a method for the treatment of muscular atrophy which comprises administering a mammal sestrin product to an animal, including a human, in need thereof.

In the sense of the present invention the term "treatment" is referred to the administration of an agent aimed to ameliorate the cause or the symptoms of muscular atrophy associated to diseases or physical conditions, wherein muscle cells are non-functional or partially deprived of function due to protein mutations, down-regulation of protein synthesis, or immobilization, but also to avoid the onset of such symptoms. Thus, for the scope of the present invention the term "treatment" also includes the concept of prevention.

Although experimental data below are performed by over-expressing mammal sestrins in a host from a vector containing the DNA codifying for these proteins, external administration of appropriate doses of these proteins in a purified form will have the same effect.

The invention has the advantage that atrophy may be rescued, compensated or avoided by promoting cell hypertrophy, and in consequence recovering muscle mass. As above indicated, muscle weight loss leads to weakening states. The weakening states may cause death, due to the immobilization of critical muscles that control, for example, the pulmonary system.

Sestrins are small, conserved proteins that accumulate in cells upon genotoxic, oxidative and metabolic stresses. Sestrins function as activators of AMP-activated protein kinase (AMPK) and inhibitors of target of rapamycin (TOR) complex 1 (TORC1).

There are three isoforms of sestrin in mammals named sesn-1, sesn-2 and sesn-3. The three of them are accumulated under energy demand conditions, such us starvation, overloading, exercise or regeneration, as well as in genotoxic, oxidative and metabolic stresses.

In human, sestrin-1 (or sesn-1), also known as p53-regulated protein PA26, is a protein that in humans is encoded by the *SESN1* gene. The amino acid sequence of sestrin-1 is the one under the UniProtKB/Swiss Prot database Q9Y6P5-1, version 2 from April 27, 2001. In the present disclosure is also referred by SEQ ID NO: 1.

The *SESN1* gene is conserved in chimpanzee, dog, cow, mouse, rat, chicken, and zebrafish.

Sestrin-2 (or sesn-2) also known as Hi95 is a protein that in humans is encoded by the *SESN2* gene. The amino acid sequence of sestrin-2 is the one under the UniProtKB/Swiss Prot database P58004, version 1 from April 27, 2001. In the present disclosure is also referred by SEQ ID NO: 2.

The *SESN2* gene is conserved in chimpanzee, dog, cow, and mouse.

In human, sestrin-3 (or sesn-3) has the amino acid sequence under the UniProtKB/Swiss Prot database P58005, sequence version of Jan 23, 2002. In the present disclosure is also referred by SEQ ID NO: 3.

In mice, sestrin-1, also known as p53-regulated protein PA26, is a protein that in mice is encoded by the *SESN1* gene. The amino acid sequence of sestrin-1 is the one under the UniProtKB/Swiss Prot database P58006, version 2 from July 5, 2005. In the present disclosure is also referred by SEQ ID NO: 4.

Mice sestrin-2 is a protein encoded by the *SESN2* gene. The amino acid sequence of sestrin-2 is the one under the UniProtKB/Swiss Prot database Q3TCT3, version 1 from October 11, 2005. In the present disclosure is also referred by SEQ ID NO: 5.

Sestrin-3 in mice is represented by the amino acid sequence under the UniProtKB/Swiss Prot database Q9CYP7, Version 1, last modified April 27, 2001. In the present disclosure is also referred by SEQ ID NO: 6.

As used herein, the term "mammal sestrin product" means a product comprising a fragment or the entire polypeptide sequence of a mammal sestrin. Suitable mammal sestrin products include natural, synthetic, or recombinant biologically active polypeptide of mammal sestrins; biologically active polypeptide variants of mammal sestrins or fragments thereof, including hybrid fusion proteins or dimers; or to biologically active polypeptide analogues of mammal sestrins or fragments thereof. Analogues include products where one or more amino acid residues have been replaced by a different amino acid. Conservative amino acid substitutions are preferred. The mammal sestrin product may be directly and externally administered as a purified polypeptide. On the other hand, using the gene therapy, the mammal sestrin product may be produced *in situ* in the treated mammal (including human) by administering a suitable vector comprising the nucleotide sequence codifying for the mammal sestrin product, said mammal sestrin product being functionally delivered into the mammal body after it has been duly expressed with the aid of the nucleotide constructs constituting the expression vector. Recombinant biologically active polypeptides of mammal sestrins can be administered as purified polypeptides derived from an expression vector or, in the alternative in the form of vectors, as such.

Functionality of the administrated sestrin product can be easily assessed by administration to a cell line (such as the myoblast cell line C2C12) and taking microscopic images from the cultures. Treated cells having a size greater than the size of the controls (not sestrin product treatment) indicates a hypertrophic phenotype.

Sestrin products may be produced by isolation and purifycation from mammal tissues. Alternatively, they can be produced by biotechnological methods including cells (microorganisms, cell lines, etc.) , which allow the obtention of the protein in the form of a recombinant protein. Said recombinant protein is derived from specialised expression nucleic acid vectors. The term "vector" in the present invention refers to a composition of matter which can be used to deliver a nucleic acid of interest to the interior of a cell. Non-limiting examples of vectors include an autonomously replicating plasmid or a virus. For purposes of this invention, the terms "expression construct," "expression vector," and "vector," are used interchangeably to demonstrate the application of the invention in a general, illustrative sense, and are not intended to limit the invention.

In Hee Lee et al., "Sestrin as Feedback Inhibitor of TOR That Prevents Age-Related Pathologies", Science - 2010, Vol. 327, pp.: 1223-1228, sestrin is indicated as a natural inhibitor of age-related myopathies in Drosophila (which has a single sestrin; dSesn). Hee Lee et al. show that dSesn-null flies exhibited highly accelerated age-related skeletal and cardiac muscle degeneration that was preceded by accumulation of swollen mitochondria, ubiquitinated protein aggregates and reactive oxygen species (ROS), caused by defective autophagy. In Drosophila it is proved that sestrin prevents pathologies caused by chronic TOR activation that may result from diminished autophagic clearance of damaged mitochondria, protein aggregates, or lipids. The authors demonstrate that over-expression of dSesn reduced the size of the cells in larval wing discs and adult eyes, although the cell number of these areas remained unchanged. They concluded that Sesn inhibits cell growth without affecting cell proliferation and does so independently of its redox activity. Additionally, Hee Lee et al. demonstrate that the expression of exogenous dSesn prevented muscle degeneration due to the maintenance of the cell proliferation rate.

Budanov et al., "p53 Target Genes Sestrin1 and Sestrin 2 Connect Genotoxic Stress and mTOR Signaling", Cell - 2008, Vol. 134, pp.: 451-460, discloses that both sestrins in HEK293 cells provoked cell size decrease.

Thus, apart of acting as agents to fight oxidative effects in cells, the teachings allow to affirm that sestrins have another effect: to diminish cell size.

Surprisingly the inventors have now found that muscle in basal condition, in which sestrins are over-expressed, behave a phenotype of cell hypertrophy, or which is the same, a pattern of enlargement of cell size. These observations are well shown in the examples below. The induction or promotion of muscle cell hypertrophy implies also the advantage of increasing muscle mass. The examples below demonstrate moreover that sestrins can rescue atrophic muscle cells. Due to this rescue ability, mammal sestrins are interesting tools as agents for the treatment of those diseases ongoing with muscle atrophy, being the diseases from different causes. Indeed and as derivable from the examples below, mammal sestrins give raise to greater mammal cells in comparison with non-treated muscles; to a greater number of fibers in the muscle; and to an increased level of regenerating muscle cells.

So then, the present invention provides a mammal sestrin product for use in the treatment of muscle atrophy.

Although the examples below are directed to skeletal mice muscle, they can be extended to cardiac muscle since both are striated muscles. Striated muscle tissue comprises muscle fibers as individual components. The fibers are combined into parallel fibers.

Thus, in the sense of the present invention "muscle" refers to multiple bundles of striated muscle tissue (skeletal or cardiac), and the term "muscle cell" and "muscle fiber" are used as synonymous and refer to a cell unit into this tissue. In particular, muscle cells, also named myofibers (muscle fiber) are long, cylindrical, multinucleated cells composed of actin and myosin myofibrils repeated as a sarcomere, the basic functional unit of the muscle fiber and responsible for skeletal muscle's striated appearance and forming the basic machinery necessary for muscle contraction. The principal cytoplasmic proteins are myosin and actin (also known as "thick" and "thin" filaments, respectively) which are arranged in a repeating unit called a sarcomere. The interaction of myosin and actin is responsible for muscle contraction.

In a preferred embodiment, the treatment of muscle atrophy by means of the use of the mammal sestrin product, is achieved by the effect of hypertrophy of cell muscles including all those forms embedded in the group of striated muscle tissue or striated muscle cells.

In a preferred embodiment the striated muscle cells are skeletal muscle cells.

In another preferred embodiment the striated muscle cells are cardiac muscle cells.

In another preferred embodiment, the mammal sestrin product for use as disclosed above, is a human sestrin. Preferably the human sestrin product is selected from the group consisting of sestrin-1 (SEQ ID NO: 1), sestrin-2 (SEQ ID NO: 2), sestrin-3 (SEQ ID NO: 3), and mixtures thereof.

In a more preferred embodiment, the sestrin product is a human sestrin-1 corresponding to SEQ ID NO: 1.

With the sestrin product for the use according to the invention, muscle atrophy which is associated to several diseases may be treated. In particular it may be treated muscle atrophy associated to a disease selected from the group consisting of muscle dystrophy, post-polio muscle atrophy, cachexia, leprosy, diabetes, cancer, renal disease, obstructive pulmonary disease, emphysema, paraplegia, hemiplegia, and sarcopenia.

Examples of muscle muscular dystrophy refer to Duchenne muscular dystrophy, Becker's muscular dystrophy, limb girdle muscular dystrophy, congenital muscular dystrophy, facioscapulohumeral muscular dystrophy, myotonic muscular dystrophy, oculopharyngeal muscular dystrophy, distal muscular dystrophy, and Emery-Dreifuss muscular dystrophy.

Among cachexias to be treated with the sestrin product, those from different aetiologies can be faced, such as cardiac disease, AIDS and cancer associated cachexia.

In another preferred embodiment of the first aspect of the invention, muscle atrophy is associated to a physical condition selected from the group consisting of malnutrition, immobilized or casted limb, and long term hospitalization.

A second aspect the invention relates to an adeno-associated vector comprising a polynucleotide sequence codifying for a mammal sestrin product. Especially suitable vectors are the adeno-associated virus encompassing the protein of interest or a fragment thereof functionally active. These vectors are specially adapted to be used in the treatment of muscle atrophy.

In general, these expression vectors usually comprise a promoter operably linked to a polynucleotide encoding the inhibitory oligonucleotide. As used herein, the terms "operably linked" or "under transcriptional control" as referred to an oligonucleotide mean that a promoter is in the correct location and orientation in relation to the oligonucleotide to control the initiation of transcription by RNA polymerase and expression of said oligonucleotide. The term "promoter" refers to a DNA sequence recognized by the synthetic machinery of the cell, or introduced synthetic machinery, required to initiate the specific transcription of an oligonucleotide.

When mammal sestrin products are used derived from an expression vector, for example by administering said vector to an individual (mammal including human), preferred adeno-associated vectors are those which comprise a human sestrin selected from the group consisting of sestrin-1, sestrin-2, sestrin-3, and mixtures thereof. Preferably, the human sestrin in the adeno-associated vector is human sestrin-1 corresponding to SEQ ID NO: 1. This adeno-associated vector is, therefore, a suitable tool for the treatment of muscle atrophy, by administering it to an animal, including a human, in need thereof. This can also be formulated as the adeno-associated vector for use in the treatment of muscle atrophy. This preferred aspect may also be formulated as the use of the adeno-associated vector comprising the sequence codifying for a mammal sestrin product for the preparation of a medicament for the treatment of muscle atrophy.

The mammal sestrin product, purified from a natural source, in the form of a recombinant protein or in the form of an expression vector can be administered as a pharmaceutical composition. In any of these forms the sestrin product is adapted to be used in the treatment of muscle atrophy.

Therefore, a third aspect of the invention is a pharmaceutical composition comprising an adeno-associated vector, as defined above, along with pharmaceutically acceptable excipients and carriers.

When administered *in vivo,* the mammal sestrin product gives raise to a phenotype of muscle cell hypertrophy, in other words, of cell size enlargement. In mice this capacity leads to animals with an increase in the volume of an organ or tissue due to this enlargement of the cells.

The pharmaceutical composition according to the invention comprising an adeno-associated vector as defined above is preferably for use in the treatment of muscle atrophy. Preferably human sestrins are employed and more preferably human sestrin-1 corresponding to SEQ ID NO: 1.

The mammal sestrin product is administered by any method known to those in the art suitable for delivery to the targeted organ, tissue, or cell type. For example, in certain embodiments of the invention, the composition may be administered by parenteral administration, such as intravenous injection, intra-arterial injection, intrapericardial injection, or subcutaneous injection, or by direct injection into the tissue (e.g., intramuscular). In other embodiments, the pharmaceutical composition that comprises a mammal sestrin product or an adeno-associated virus comprising it, may be administered by oral, transdermal, intraperitoneal, subcutaneous, sustained release, controlled release, delayed release, suppository, or sublingual routes of administration. The composition of the invention may also be administered by a catheter system.

In a preferred embodiment, the pharmaceutical composition of the invention is administered intramuscularly, and is thus formulated in a form suitable for intra-muscular administration. Suitable formulations for intra-muscular administration include solutions, suspensions, dispersions, emulsions, oils and the like.

The pharmaceutical compositions of the invention are formulated in conjunction with pharmaceutically acceptable excipients and/or carriers. The term "pharmaceutically acceptable" as used herein refers to compounds, materials, compositions, and/or dosage forms which are, within the scope of sound medical judgement, suitable for being administered to an animal, including a human, without undue toxicity, incompatibility, instability and allergic response, among others. Each pharmaceutically acceptable carrier, excipients, etc., must also be acceptable in the sense of being compatible with the other ingredients of the pharmaceutical formulation.

Pharmaceutically acceptable excipients and/or carriers used in the composition of the present invention are specifically suitable for gene therapy and preferably designed to enhance the stability and bioavailability of the mammal sestrin products.

In one embodiment, the composition of the invention comprises excipients and/or vehicles suitable for parental, preferably, intramuscular administration. Parenteral vehicles include sodium chloride solution, Ringer's dextrose, dextrose and sodium chloride, lactated Ringer's and fixed oils. Intravenous vehicles include fluid and nutrient replenishers, electrolyte replenishers such as those based on Ringer's dextrose, and the like. Examples are sterile liquids such as water and oils, with or without the addition of a surfactant and other pharmaceutically acceptable adjuvants. In general, water, saline, aqueous dextrose and related sugar solutions, and glycols such as propylene glycols or polyethylene glycol are preferred liquid carriers, particularly for injectable solutions.

The pharmaceutical compositions provided herewith may be controlled-release compositions, i.e. compositions in which the active compound is released over a period of time after administration. For example, the agent may be administered using intravenous infusion, an implantable osmotic pump, a transdermal patch, liposomes, or other modes of administration. Alternatively, the composition is an immediate-release composition, i.e. a composition in which of the active compound is released immediately after administration.

The compositions also include, in another embodiment, incorporation of the active material into or onto particulate preparations of polymeric compounds such as polylactic acid, polglycolic acid, hydrogels, etc, or onto liposomes, microemulsions, micelles, unilamellar or multilamellar vesicles, erythrocyte ghosts, or spheroplasts.) Such compositions will influence the physical state, solubility, stability, rate *of in vivo* release, and rate *of in vivo* clearance. Liposomes including the mammal sestrin product or adeno-associated vector comprising it, are particularly suitable for the delivery of these products for use in the treatment of muscle atrophy according to the present invention.

In one embodiment, the present invention comprises the use of the mammal sestrin product or adeno-associated vector comprising it, as a sole active ingredient for the treatment of muscle atrophy. However, also encompassed within the scope of the present invention is the use of these compounds in combination with one or more additional therapeutic agents. In a particular embodiment, these additional agents are appropriate for treating muscle atrophy. As will be apparent to the skilled in the art, the combination of a mammal sestrin product or adeno-associated vector comprising it, with one or more additional active ingredients, may be effective not only when the active ingredients are used in a single pharmaceutical composition, but also when used in two different compositions, either administered simultaneously, sequentially or separately after a certain period of time.

In a further aspect, the invention relates to a non-human animal model of muscle cell hypertrophy obtainable by administering to said animal an external amount of a mammal sestrin product or an adeno-associated vector, both as defined above, wherein said mammal has a phenotype of muscle cell hypertrophy.

In a preferred embodiment of the non-human animal model of muscle cell hypertrophy, the mammal sestrins are over-expressed when administered by means of an adeno-associated vector comprising them. Preferably, the non-human animal is a mouse, wherein in a period between 20 and 40 days after the administration of the mammal sestrin product, clear hypertrophic muscles are observed.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### BRIEF DESCRIPTION OF THE DRAWINGS

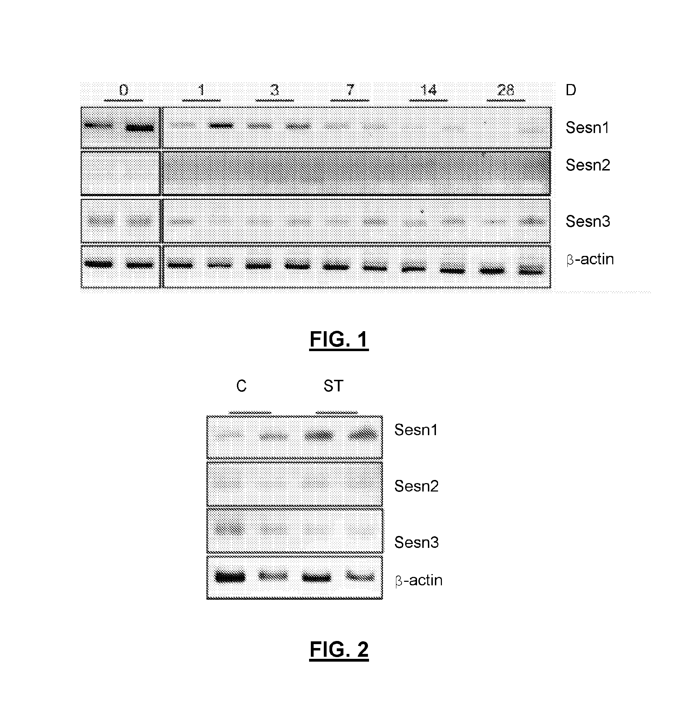

FIG. 1, related to results 1, is an agarose gel electrophoresis image showing the levels of cDNA from transcripts (mRNA) of sestrin-1 gene (sesn1), sestrin-2 gene (sesn2) and sestrin-3 gene (sesn3). D means days post-denervation. β-actin is the positive electrophoresis control.
FIG. 2, related to results 1, is also an agarose gel electrophoresis image showing the levels of cDNA from transcripts (mRNA) of sestrin-1 gene (sesn1), sestrin-2 gene (sesn2) and sestrin-3 gene (sesn3) after 24 hours of starvation. β-actin is the positive electrophoresis control. C means control mice; ST means starved mice.

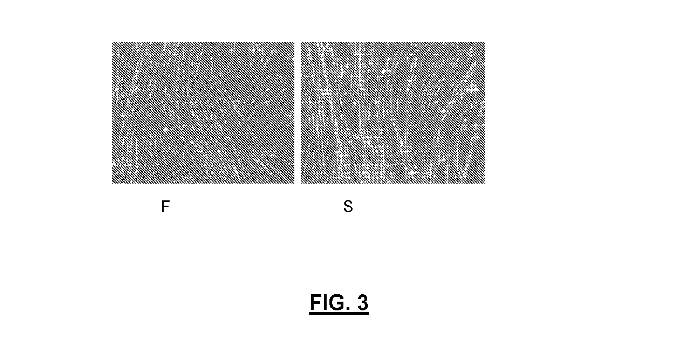

FIG. 3, related to results 2, is an image taken by optical miscroscopy (microscope DMR6000B (Leica) equipped with a DFC300FX camera) from the myoblasts cell line called C2C12 in fed and starved conditions. F means fed; and ST means starved.

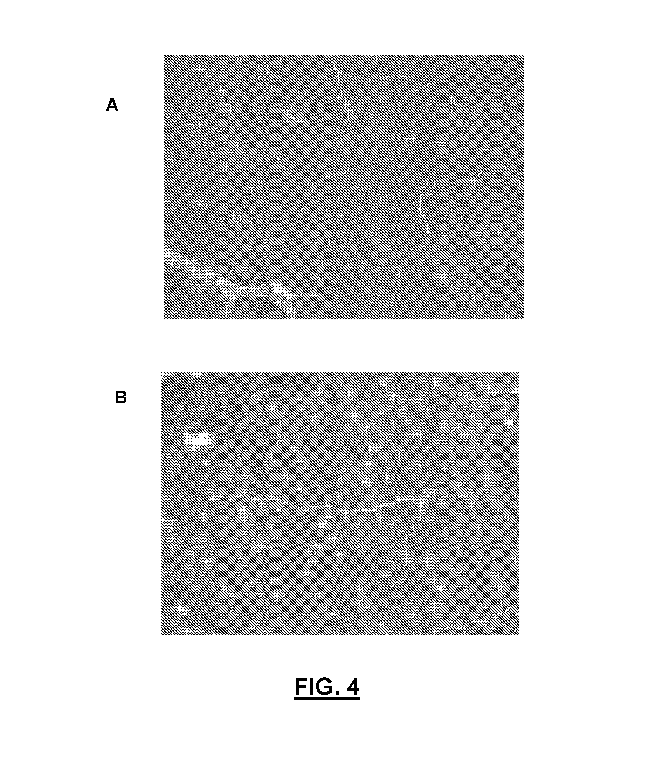

FIG. 4, related to results 3, corresponds to Digital images of the cells extracted from *tibialis* acquired with a microscope DMR6000B (Leica) equipped with a DFC300FX camera for immunohistochemical color pictures, using Plan Fluo 10×/0.3 and 20x/0.75 objective lens and maximum optical sections. Images were composed and edited in Photoshop CS4 (Adobe). Panel A is the control and corresponds to the cells of a control mouse. Panel B corresponds to the state of cells in a mouse 28 days after having received an injection of adeno-associated virus carrying the gene codifying for human sestrin-1 (AAV-Sesn1).

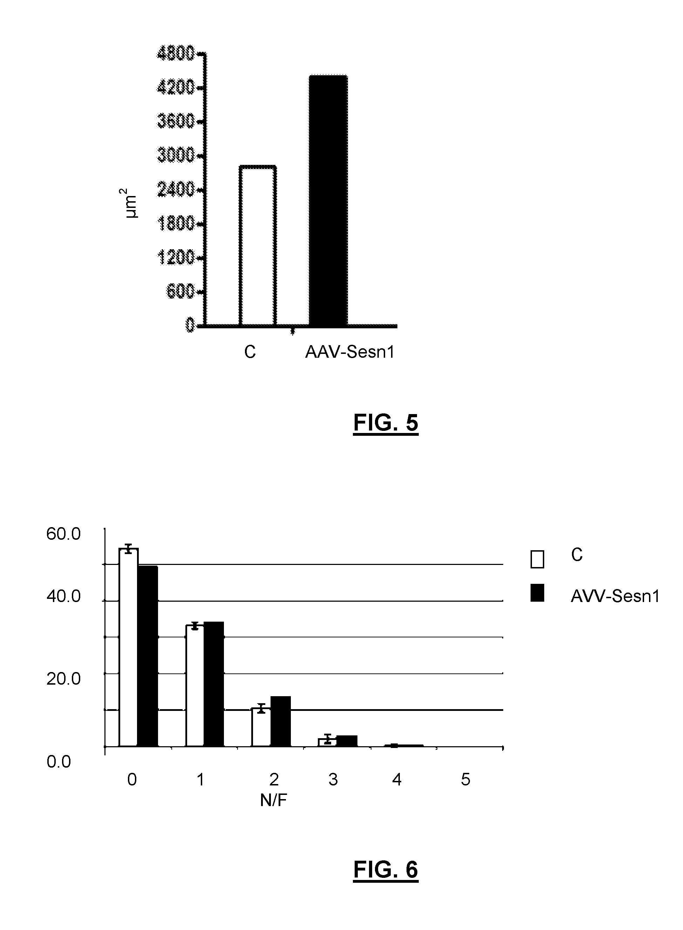

FIG. 5, related to results 3, is a bar diagram showing the number of muscle fibers per area in control (C) and tested or injected mice (AW-Sesn1) at 28 days post-injection.
FIG. 6, also relating to results 3, is a diagram bar that shows the number of nucleus per fiber (N/F) in muscles of control (C or white bars) and of injected mice with AAV-Sesn1 (AAV-Sesn1 or black bars). Y-axis shows the number of cells.

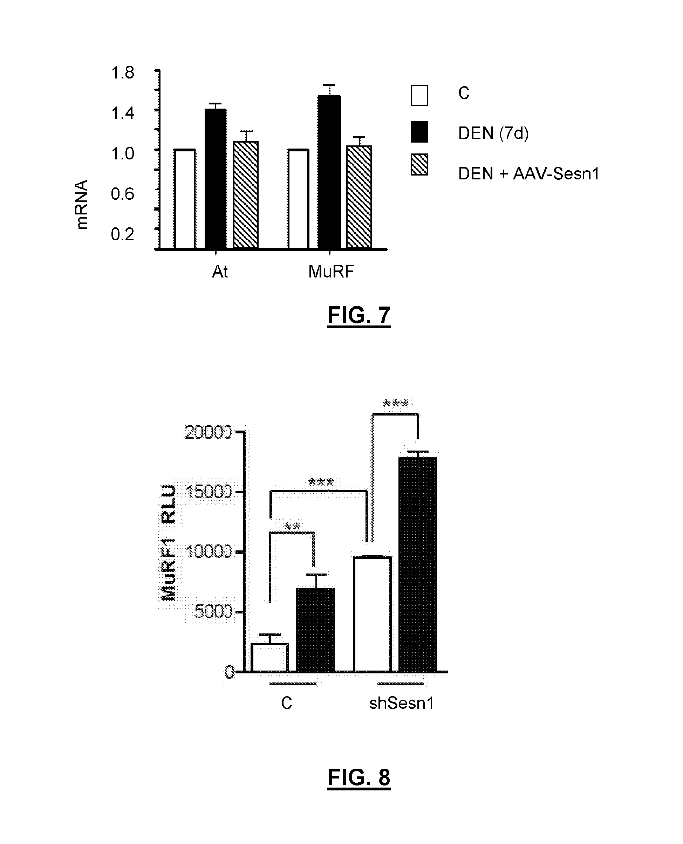

FIG. 7, related to results 4, is another diagram bar that shows the relative mRNA expression of Atrogin (At) and of the E3 ubiquitin ligase MuRF1 (MuRF), both being markers of an atrophiated tissue. White bars are the control (C); Black bars (DEN 7d) correspond to denervated mice at 7 days post-denervation; and dashed bars (DENN + AAV-hSesn1) correspond to the denervated mice which further received the treatment with AAV-Sesn1.
FIG. 8, related to results 5 is a diagram bar that shows the effect of depleting sestrin-1 expression. The effects are represented by determining the levels of expression of MuRF1 labeled with luciferase (MuRF1 RLU is relative luciferase units to detect MuRF1 levels). C means control and corresponds to mice in which no small-hairpin RNA (shRNA) was electroporated (injected). sh-sesn1 corresponds to the group of mice receiving a small-hairpin RNA depleting the transcript of sestrin 1. White bars are fed animals and black bars are fasted (24 h starvation) animals.

### DETAILED DESCRIPTION OF PARTICULAR EMBODIMENTS / EXAMPLES

The following examples are provided by way of illustration, and are not intended to be limiting of the present invention.

### Materials and methods

### 1. Animal model.

Wild-type (C57BL/6) were purchased from The Jackson Laboratory (Bar Harbor, ME, USA). Mice were housed in the animal facility of the PRBB Barcelona under conventional conditions with constant temperature and humidity and fed a standard diet. Male C57BL/6mice, aged 5-6 weeks, were used for AAV injections, starvation and denervation experiments. Starvation experiments were done by fasting one group of mice for 24 hours before sacrifice.

Denervation was produced by cutting the sciatic nerve in another group of mice high in the thigh after the every mouse had been anesthetized with ketamine/xylazine (80/10 mg/kg).

All animal experiments were approved by the Catalan Government Animal Care Committee.

### 2. Preparation of adeno-associated virus comprising sestrin mammal products and in vivo administration.

AAV2/1-CMV-GFP and AAV2/1-CMV-Sesn1-IRES-GFP were produced in the Centre for Animal Biotechnology and Gene Therapy of the Universitat Autonoma of Barcelona, (CBATEG-UAB). GFP means Green Fluorescent Protein; CMV is an enhancer derived from cytomegalovirus; IRES means internal ribosome entry site.

AAV vector construction, production and titration for AAV2/1-CMV-Sesn1-IRES-GFP and its control AAV2/1-sh(-)-IRES-GFP:

Throughout the disclosure the vector constructions AAV2/1-CMV-Sesn1-IRES-GFP and AAV2/1-sh(-)-IRES-GFP are mentioned in a simplified mode as AAV-Sesn1 and control (C), respectively.

cDNA corresponding to SEQ ID NO: 1 (human sestrin-1) was cloned into Xhol and BamHI sites between the internal terminal repeats of adeno-associated virus serotype 2 (AAV2), under the regulation of the chicken β-actin promoter and the enhancer (CAG) of cytomegalovirus (CMV). The woodchuck hepatitis virus responsive element was added at the 3' end to stabilize mRNA expression, as disclosed in Loeb JE et al, "Enhanced expression of transgenes from adeno-associated virus vectors with the woodchuck hepatitis virus posttranscriptional regulatory element: implications for gene therapy". Hum Gene Ther-1999, Vol. 10, pp.: 2295-2305.

AAV2/1-CMV-Sesn1-IRES-GFP and AAV2/1-sh(-)-IRES-GFP were generated as previously described by Zolotukhin S et al., "Recombinant adeno-associated virus purification using novel methods improves infectious titer and yield", Gene Therapy -1999, Vol. 6, pp.: 973-985. Briefly, the vectors were generated by triple transfection of HEK 293-AAV cells (Stratagene, Carlsbad, CA, USA) with branched polyethylenimine (Sigma, St Louis, MO, USA), with the plasmid containing the internal terminal repeats of AAV2, the AAV-helper plasmid for each serotype (kindly provided by JM Wilson, University of Pennsylvania, Philadelphia, PA, USA), and the pXX6 plasmid containing helper adenoviral genes. The technology using pXX6 plasmid is disclosed and directly applicable from Xiao X, Li J, Samulski RJ. Production of high-titer recombinant adeno-associated virus vectors in the absence of helper adenovirus. J Virol -1998, Vol. 72, pp.: 2224-2232.

Vectors were purified by CsCl or iodixianol gradients. Encapsidated DNA was quantified by a PicoGreen assay (Invitrogen, Carlsbad, CA, USA) following denaturation of the AAV particles and the titers were calculated as µg per ml. Titers for adeno-associated viruses encompassing GFP (rAAVGFP vectors, or which is the same: AAV2/1-CMV-Sesn1-IRES-GFP and AAV2/1-sh(-)-IRES-GFP) in international units (IU) per ml were measured in QBI-HEK 293A cells (Q-Biogene, Carlsbad, CA, USA) by counting transduction events 72 h after vector exposure.

Small skin incisions were made in the targeted muscle areas of health mice (Wild-type (C57BL/6)), after every mouse had been anesthetized. The corresponding vector was injected into the *tibialis* (40 µl, 5E+12 gc/ml) and *soleus* (25 µl, 5E+12 gc/ml) of each leg using a Hamilton syringe. Gc/ml are genome copies per millilitre.

### 3. In vivo transfection of shRNA and luciferase assays.

Adult health mice muscles were transfected by intramuscular injection of small-hairpin RNA (shRNA), 20 µg total, followed by electroporation to increase gene transfer efficiency. Electric pulses were applied by two stainless steel spatula electrodes connected to an ECM830 BTX porator (Genetronics, San Diego, CA, USA) with the following settings: 5 pulses of 20 ms each and 200 ms interval, the voltage was adjusted according to the thickness of the muscle (220 V cm-1). Muscles were analyzed 8 days later (with or without a 24 hour fasting period). Muscles were collected and luciferase activity was determined with a luciferase assay kit (Promega). Activity results were normalized by dividing luciferase activity by β-galactosidase activity, this later analysed by any of the known methods, such as the colorimetric assay with the substrate ortho-Nitrophenyl-β-galactoside (ONPG).

### 4. RNA isolation and quantitative real time PCR (qPCR).

Total RNA was isolated from muscle tissue using miRNeasy Mini Kit (Qiagen). For qPCR experiments (when required), DNase digestion of 10 mg of RNA was performed using 2 U DNase (Turbo DNA-free, Ambion). Complementary DNA was synthesized from 2 mg of total RNA using the First-Strand cDNA Synthesis kit (Amersham Biosciences). For qPCR, reactions were performed on a LightCycler 480 System using a Light Cycler 480 SYBR Green I Master (Roche Diagnostic Corporation) and specifics primers. Thermocycling conditions were as follow: initial step of 10 min at 95 °C, then 50 cycles of 15 s denaturation at 94 °C, 10 s annealing at 60 °C and 15 s extension at 72 °C. Reactions were run in triplicate, and automatically detected threshold cycle (Ct) values were compared between samples. Transcripts of the ribosomal protein L7 gene were used as endogenous control, with each unknown sample normalized to L7 content.

### 5. Western blotting.

Cell lysates were prepared with IP buffer (50 mM Tris HCL pH 7.5, 150 mM NaCl, 1% NP-40, 5 mM EGTA, 5 mM EDTA, 20 mM NaF, 25 mM β-glycerophosphate, 0.1 mM sodium vanadate, 1 mM PMSF) supplemented with protease and phosphatase inhibitors (Complete Mini, Roche Diagnostic Corporation; phosphatase inhibitor cocktail, Sigma). Cleared lysates containing approximately 50 µg of protein were separated by SDS-PAGE and transferred to nitrocellulose membranes.

### 6. Histology and immunohistochemistry.

*Tibialis* muscles were frozen in isopentane cooled with liquid nitrogen, and stored at -80 °C until analysis. Cryosections (10 µm thickness) were stained with Hematoxilin/eosin (H/E, SIGMA) or Sirius Red (Sigma). Digital images were acquired with a microscope DMR6000B (Leica) equipped with a DFC300FX camera for immunohistochemical color pictures, using Plan Fluo 10×/0.3 and 20x/0.75 objective lens and maximum optical sections. Images were composed and edited in Photoshop CS4 (Adobe), where background was reduced using brightness and contrast adjustments applied to the whole image. Image analysis of hypertrophied tissue was performed with the public domain computer-assisted image analysis software ImageJ (NIH, USA). Fiber size (area) measurements were performed as described in- Suelves M, et al. "uPA deficiency exacerbates muscular dystrophy in MDX mice". J Cell Biol 2007, vol. 178, p. 1039-1051)..

### 7. Cell culture for biochemical analysis.

The C2C12 cell line was cultured in DMEM containing 10% FCS, 100 U/ml penicillin, 100 µg/ml streptomycin, 0.001% Fungizone, (GM growth medium). To induce differentiation in C2C12 myoblasts, GM was replaced by differentiation medium DM (DMEM supplemented with 2% horse serum, 2 mM Lglutamine, 100 U/ml penicillin, 100 µg/ml streptomycin and 0.001% Fungizone) at myoblast subconfluence. To induce starvation in C2C12 myotubes, C2C12 cells were left in DM for 4 days and substituted for Hank's Balanced Salt Solution (HBSS) for the indicated time before samples were collected.

### 8. Statistical analysis.

Graphpad Prism software was used for all statistical analyses. Results from corresponding time points of each group were averaged and used to calculate descriptive statistics. One-way ANOVA and Tukey post-hoc test, or Dunnett's post-hoc test were used on multiple comparisons and all possible pair wise comparisons among groups at each time point. Data are mean ± s.e.m. Significance was accepted at P≤0.05.

### Results

The following results demonstrate that mammal sestrins are involved in the regulation of the muscle mass in atrophic processes by means of the induction of cell hypertrophy.

### 1. Denervation and starvation produce alterations of sestrin levels in mice.

Denervated mice and starved mice showed a biochemical pattern wherein the levels of different mice sestrin types (sestrin-1, 2 and 3) were affected by the denervation and starvation processes. To the best of the applicant's knowledge, this is the first time that sestrin levels in such a situation have been analyzed in mice.

FIG. 1 corresponds to an image of an electrophoresis in agarose gel of the cDNA derived from mice muscle *(tibialis)* cell lysates analyzed after denervation. As can be seen, sestrin levels were significantly reduced from day 0 to day 28 post-denervation, with the exception of sestrin-3 levels. It is known that denervation produce muscle atrophy. Sestrin-1 levels decreased progressively. On the other hand sestrin-2 levels decreased drastically. Finally, sestrin-3 levels were progressively increased.

On the other hand, the starvation process, which results are depicted in FIG. 2, show that the levels of mRNA (cDNA) of sestrin-1 and sestrin-2 were increased in starved versus control mice. Levels of sestrin-3 remained unchanged. β-actin is the positive electrophoresis control. Without being bound to the theory, the inventors hypothesize that sestrin-1 levels in starved mice are increased as a protection cell mechanism. Probably, a prolongued starvation will progress to a decrease in the levels of the protein.

For the analysis of sestrin levels, both in starvation and denervation assays, total RNA was isolated from muscle tissue using miRNeasy Mini Kit (Qiagen). For qPCR experiments, DNase digestion of 10 mg of RNA was performed using 2 U DNase (Turbo DNA-free, Ambion). Complementary DNA was synthesized from 2 mg of total RNA using the First-Strand cDNA Synthesis kit (Amersham Biosciences). As positive control of the electrophoresis β-actin cDNA was detected. For the detection of the different sestrin types and the respective levels, ethidium bromide was used and the gel was visualized.

### 2. Starved myoblasts show atrophic phenotype

As can be seen in FIG. 3, starved cells (C2C12) showed an atrophic phenotype defined as longer and thinner shape, meanwhile fed cells were in a health state.

### 3. In vivo administration of AAV-Sesn1.

Adeno-associated virus with the gene codifying for human sestrin-1, corresponding to SEQ ID NO: 1 (AAV-Sesn1) were prepared and administered (single injection) to health (Wild-type (C57BL/6)) mice as indicated in methods 2. Control mice (C) were injected with the adeno-associated virus without the gene for sestrin-1 (AAV2/1-sh(-)-IRES-GFP).

28 days later, a sample of the *tibialis* of control and injected mice were extracted and processed as indicated in methods 6.

FIG. 4, panels A and B, show that in injected mice muscle cells were greater than those of the control, thus showing a hypertrophic phenotype.

FIG. 5 is an additional experiment, showing the number of fibers detected per area of studied muscle (fibers/ µm²). This parameter was calculated as exposed in methods 6. As can be seen the number of fibers in injected mice (AAV-Sesn1) (N=2) at day 28 post-injection was significantly greater than the number in control (C) mice. This observation also conforms that the tissue in injected mice was hypertrophied.

The inventors also determined the number of nucleus per fiber (N/F) of muscle cells in injected (AAV-Sesn1) and control (C) mice. This parameter indirectly reveals the number of regenerating cells existing in a determined tissue, in such a way that the greater the number of nucleus, the greater the number of cells allowing to regenerated damaged or normal tissue.

Thus, as can be derived from FIG. 6, the number of nucleus per fiber was greater in mice which previously (28 days before) received the adeno-associated vector comprising the human gene of sestrin-1. These data allow deducing that a greater proportion of regenerating cells is present in mice treated with AAV-Sesn1 in comparison with control mice.

All these observations lead to the conclusion that mammal sestrins administered to mice promote hypertrophied tissue. Therefore, it is confirmed that mammal sestrins are useful in the treatment of muscle atrophy, since they are able to induce or promote muscle cell hypertrophy.

### 4. Mammal sestrins are able to rescue atrophied tissue.

The inventors also tested a model of atrophied tissue (denervation) in order to finally confirm that the administration of mammal sestrins were able to revert this situation due to its ability of inducing hypertrophy when over-expressed in mice.

Thus, they proceed to perform denervation as indicated in methods 1 to mice (DEN) and 7 days post-denervation they detected the levels of relative mRNA expression of two atrophic markers: Atrogin (At) and the E3 ubiquitin ligase MuRF1 (MuRF). After this, denervated mice received and injection of AAV-Sesn1 (adeno-associated virus with the human sestrin-1 gene; DEN + AAV-Sesn1). The levels of At and MuRF were detected again. As control (C), mice without denervation and without AVV-Sesn1 administration were used.

As can be seen in FIG. 7, the levels of relative mRNA expression of two Atrogin (At) and the E3 ubiquitin ligase MuRF1 (MuRF) were highly increased after denervation was done. Interestingly, after the administration of AAV-Sesn1 the levels of both atrophic markers were highly reduced, coming back to control (C) values, thus to normal levels. Thus mammal sestrins exogenously administered are able to rescue atrophic tissue.

mRNA levels were measured as indicated in methods 4.

All these experiments as a whole allow concluding that mammal sestrins are useful tools for the treatment of muscle atrophy, said muscle atrophy being associated to a disease selected from the group consisting of muscular dystrophy, post-polio muscle atrophy, cachexia, leprosy, diabetes, cancer, renal disease, obstructive pulmonary disease, emphysema, paraplegia, hemiplegia, and sarcopenia. In the same way, they are useful tools for the treatment of muscle atrophy derived from a physical condition such as a malnutrition state, the atrophy derived from an immobilized limb, or the atrophy of muscle mass due to a long term hospitalization.

### 5. Sestrin in mammals is related with atrophic patterns.

The inventors, also with the aim of resolving the possible role of mammal sestrins in atrophy, tested if fed and starved mice showed an atrophied phenotype once mouse sestrin-1 was kidnapped.

They used a small-hairpin RNA (sh-RNA) corresponding to SEQ ID NO: 7 (gttcaagcactctgagaaggt) to deplete mouse Sesn1 transcript.

As indicated in methods 3, adult mice muscles were transfected by intramuscular injection of the small-hairpin RNAs followed by electroporation to increase gene transfer efficiency. Electric pulses were applied by two stainless steel spatula electrodes connected to an ECM830 BTX porator (Genetronics, San Diego, CA, USA) with the following settings: 5 pulses of 20ms each and 200 ms interval, the voltage was adjusted according to the thickness of the muscle (220 V cm-1). Muscles were analyzed 8 days later (with or without a 24 hour fasting period). Muscles were collected and luciferase activity was determined with a luciferase assay kit (Promega). Activity results were normalized by dividing luciferase activity by β-galactosidase activity.

As can be seen in FIG. 8 fed (white bars) and fasted (black bars) showed an increase in the levels of MuRF1 once sestrin-1 had been kidnapped or (in the control group), once starvation was induced. Thus, inhibition of sestrin-1 promotes atrophied tissue.

FIG. 8 also shows that in mice in which a small-hairpin RNA for sestrin-1 (sh-Sesn) was electroporated the levels of the atrophic marker were greater in fed as well as in starved conditions.

Thus, without being bound to theory, the inventors propose that in mammals sestrins play a crucial role in the control of muscle mass and in the maintenance of normal size cells, since when they are depleted (i.e.: by kidnapping) muscle cells *in vivo* showed an atrophic phenotype.

It is also of great interest to mention that the proposed non-human animal model of muscle cell hypertrophy is a good model to study not only the pathways and processes involved in hypertrophy, but also a useful tool to test putative hypertrophy inhibitors. The hypertrophy model is a good approach, since it is a clean model generated from normal animals and avoids background from any pathology.

### REFERENCES CITED IN THE APPLICATION

- Rüegg et al., "Molecular Mechanisms and Treatment Options for Muscle Wasting Diseases", Annu. Rev. Pharmacol. Toxicol - 2011, Vol. N° 51, pp.: 373-395.
- In Hee Lee et al., "Sestrin as Feedback Inhibitor of TOR That Prevents Age-Related Pathologies", Science - 2010, Vol. 327, pp.: 1223-1228
- Budanov et al., "p53 Target Genes Sestrin1 and Sestrin 2 Connect Genotoxic Stress and mTOR Signaling", Cell - 2008, Vol. 134, pp.: 451-460.
- Suelves M, et al. "uPA deficiency exacerbates muscular dystrophy in MDX mice". J Cell Biol 2007, vol. 178, p. 1039-1051).
- Loeb JE et al, "Enhanced expression of transgenes from adeno-associated virus vectors with the woodchuck hepatitis virus posttranscriptional regulatory element: implications for gene therapy". Hum Gene Ther-1999, Vol. 10, pp.: 2295-2305.
- Zolotukhin S et al., "Recombinant adeno-associated virus purification using novel methods improves infectious titer and yield", Gene Therapy -1999, Vol. 6, pp.: 973-985.
- Xiao X, Li J, Samulski RJ. Production of high-titer recombinant adeno-associated virus vectors in the absence of helper adenovirus. J Virol -1998, Vol. 72, pp.: 2224-2232.

## Claims

1. A mammal sestrin product for use in the treatment of muscle atrophy.

2. The mammal sestrin product for use according to claim 1, wherein the treatment comprises the hypertrophy of striated cell muscles.

3. The mammal sestrin product for use according to any of the claims 1-2, wherein the sestrin is a human sestrin.

4. The mammal sestrin product for use according to claim 3, wherein the sestrin product is selected from the group consisting of sestrin-1, sestrin-2, sestrin-3, and mixtures thereof.

5. The mammal sestrin product for use according to claim 4, wherein the sestrin product is the human sestrin-1 corresponding to SEQ ID NO: 1.

6. The mammal sestrin product for use according to any of the claims 1-5, wherein the muscle atrophy is associated to a disease selected from the group consisting of muscular dystrophy, post-polio muscle atrophy, cachexia, leprosy, diabetes, cancer, renal disease, obstructive pulmonary disease, emphysema, paraplegia, hemiplegia, and sarcopenia.

7. The mammal sestrin product for use according to any of the claims 1-5, wherein the muscle atrophy is associated to a physical condition selected from the group consisting of malnutrition, immobilized or casted limb, and long term hospitalization.

8. An adeno-associated vector comprising a polynucleotide sequence codifying for a mammal sestrin product.

9. The adeno-associated vector according to claim 8, wherein the sestrin product is a human sestrin product.

10. The adeno-associated vector according to claim 9, wherein the human sestrin product is selected from the group consisting of sestrin-1, sestrin-2, sestrin-3, and mixtures thereof.

11. The adeno-associated vector according to claim 10, wherein the sestrin product is the human sestrin-1 corresponding to SEQ ID NO: 1.

12. The adeno-associated vector according to any of the claims 8-11, for use in the treatment of muscle atrophy.

13. A pharmaceutical composition comprising an adeno-associated vector as defined in any of the claims 8-12 along with pharmaceutically acceptable excipients and carriers.

14. The pharmaceutical composition according to claim 13, for use in the treatment of muscle atrophy.

15. The pharmaceutical composition according to any of the claims 12-13, which is for parenteral administration.
